# FASCICULE DE BREVET EUROPEEN

(11) **EP 3 727 594 B1**
(45) Date de publication et mention de la délivrance du brevet: **26.01.2022**
(21) Numéro de dépôt: 18845300.5
(22) Date de dépôt: 21.12.2018
(51) Int. Cl.: A61Q 1/02, A61K 8/73, A61Q 19/00, A61K 8/99, A61K 36/00

(54) **COMPOSITION COSMÉTIQUE COMPRENANT UN EXTRAIT DE CAESALPINIA SPINOSA, UN EXTRAIT DE KAPPAPHYCUS ALVAREZII, AU MOINS UN PRÉBIOTIQUE ET UN PROBIOTIQUE**
KOSMETISCHE ZUSAMMENSETZUNG MIT EINEM CAESALPINIA-SPINOSA-EXTRAKT, EINEM KAPPAPHYCUS-ALVAREZII-EXTRAKT, MINDESTENS EINEM PRÄBIOTIKUM UND EINEM PROBIOTIKUM
COSMETIC COMPOSITION COMPRISING A CAESALPINIA SPINOSA EXTRACT, A KAPPAPHYCUS ALVAREZII EXTRACT, AT LEAST ONE PREBIOTIC AND A PROBIOTIC

(30) Priorité: 22.12.2017 FR 1763139
(43) Date de publication de la demande: 28.10.2020
(73) Titulaire: L V M H RECHERCHE, 45800 St. Jean de Braye (FR)
(72) Inventeur: DUMAS, Marc, 45650 SAINT JEAN LE BLANC (FR); NOTTE, David, 45000 ORLEANS (FR); PELLICIER, Françoise, 45470 LOURY (FR)
(74) Mandataire: Regimbeau
(86) Numéro de dépôt international: PCT/FR2018/053502
(87) Numéro de publication internationale: WO 2019/122776

(56) Documents cités:
- WO-A1-2017/063066

## Description

### DOMAINE DE L'INVENTION

La présente invention concerne une composition cosmétique comprenant un agent cosmétique constitué par des galactomannanes et des galactanes sulfatés réticulés, et au moins un prébiotique et un probiotique, et son utilisation notamment pour maintenir l'équilibre du microbiote cutané et favoriser et/ou améliorer la tenue du maquillage.

### ETAT DE LA TECHNIQUE

La peau, plus grand organe humain, est colonisée par des milliards de micro-organismes (bactéries, levures, champignons, virus...) collectivement appelés 'microbiote' ou 'microflore'. Le terme 'microbiome cutané' désigne quant à lui l'ensemble de ces micro-organismes, leur génome et leurs interactions avec leur environnement.

Le nombre de bactéries présentes sur la peau peut atteindre des millions par cm².

La flore cutanée humaine peut être subdivisée en deux groupes :
- La flore transitoire composée de champignons, virus et bactéries pour la plupart inoffensives, dites saprophytes, qui se nourrissent de matières organiques en décomposition provenant de l'environnement. Cette flore n'est pas permanente, elle varie dans la journée, selon les activités réalisées et les variations des conditions environnantes et l'exposition des individus à ces conditions. Les espèces transitoires les plus communes sont *Staphylococcus aureus, Escherichia coli, Pseudomonas aeruginosa* et des espèces *de Bacillus* ;
- La flore résidente est composée de germes commensaux, c'est-à-dire vivants grâce à leur hôte sans lui causer de dommage. La composition de cette flore est fixe, et après perturbations, ses mêmes composants se reforment spontanément. Ces micro-organismes (bactéries Gram positif, Gram négatif...) habitent l'épiderme et se retrouvent principalement dans les couches supérieures du *stratum corneum* ainsi que dans les conduits des glandes sudoripares et des follicules pilo-sébacés et les annexes épidermiques que sont les ongles et les cheveux.

Ces micro-organismes sont indispensables à la vie. Outre leur rôle dans l'apparition des odeurs corporelles, ils sont en effet étroitement liés au maintien d'un bon état de santé de la peau. Les microbes résidents et transitoires ne causent ni maladie ni dysfonctionnement dans des conditions normales, c'est-à-dire lorsqu'une hygiène adéquate est assurée et lorsque la flore résidente, les réponses immunitaires et la fonction barrière de la peau sont intactes. Le microbiote cutané est ainsi capable de jouer un rôle de barrière et de protéger son hôte.

En parallèle, l'épiderme génère des lipides et des peptides antimicrobiens, tels que les β-défensines, des récepteurs dédiés à la reconnaissance des pathogènes qui, tous ensemble forment 'l'immunité innée cutanée'.

En se développant et en se multipliant, les bactéries résidentes produisent par ailleurs des métabolites toxiques, les bactériocines, et des sérines protéases comme celles de *Staphylococcus epidermidis* permettant d'inhiber la croissance des autres micro-organismes et d'empêcher la formation de leur biofilm par lequel ils adhèrent à la peau..

La flore commensale stimule la barrière cutanée et l'immunité innée permettent ainsi le maintien d'une peau en bonne santé et résistante. L'équilibre du microbiote de la peau, ainsi que l'expression des conditions écologiques du milieu cutané (température, pH, salinité, teneurs hormonales, teneur en lipides et en protéines, en eau et en oxygène, exposition aux UV) sont également essentiels à ce maintien.

Outre les facteurs intrinsèques tels que l'âge, le sexe, la constitution génétique et la réactivité immunitaire, les facteurs extrinsèques tels que le climat (température, humidité ambiante, UV), les traitements médicamenteux, l'utilisation de produits cosmétiques d'hygiène, de soin ou de maquillage peuvent avoir un impact important sur la composition des communautés microbiennes cutanées.

Il a également été démontré que la composition du microbiote cutané est influencée par le la capacité de la peau à retenir l'eau qu'elle contient et la production de sébum. Le niveau de sébum est corrélé négativement avec les 3 indices de diversités à partir de données taxonomiques 16S (ordre, famille, genre et espèces) et la même tendance a été observée pour une perte insensible en eau (reflétant la qualité de la barrière cutanée).

Ces études présentées au Congrès de l'ASCB de San Francisco en décembre 2016 par C Heusèle et al. et intitulé 'Microbiota disversity on healthy women's face in relaton to skin biophysical characteristics', mettent en évidence une corrélation entre la production de sébum et la fonction barrière de la peau avec la composition et la diversité du microbiote.

La variabilité du microbiote cutané humain peut alors entraîner une altération de sa structure (dysbiose) et avoir des conséquences sur la santé en engendrant la survenue de dysfonctionnements cutanés ou contribuer au développement de pathologies via la colonisation et la prolifération de la flore résidente et/ou transitoire. Par exemple on observe une diminution de la diversité microbienne dans certaines pathologies comme la dermatite atopique (Flores G et al. J Drugs Dermatol 2014: 13(11): 1365-72) et le psoriasis (Alekseyenko A et al., Microbiome 2013 : 1(131) : 2-17)

L'application en particulier de compositions de maquillage, telles que des fonds de teint, peut ainsi perturber l'équilibre du microbiote cutané, en particulier en perturbant la diversité bactérienne. Le film invisible de maquillage appliqué sur la peau peut également générer transitoirement une modification de l'équilibre hydrolipidique de la peau et de la desquamation, facteurs susceptibles de déréguler l'équilibre du microbiote cutané.

Le film invisible de maquillage appliqué sur la peau peut aussi générer transitoirement une condition d'hypoxie susceptible de modifier le microbiote cutané, en particulier les microorganismes aérobies qui vivent à la surface du tégument où la teneur en oxygène est la plus élevée. Il a en effet été mis en évidence qu'une application de fond de teint impactait significativement la diversité bactérienne (Staudinger T et al. J Appl. Microbiol 2011 : 110 (6) : 1381-9.

Le film lipidique constitué par une production excessive de sébum peut également générer transitoirement une condition d'hypoxie susceptible de modifier le microbiote cutané, en particulier les microorganismes aérobies qui vivent à la surface du tégument où la teneur en oxygène est la plus élevée.

Mais l'hypoxie est également capable d'altérer le tissu cutané lui-même à plusieurs niveaux :
- en réduisant la formation de desmogleine-1 une protéine constitutive des liens de cohésion entre les cellules et donc de la résistance de l'épiderme (Straseski J et al. Wound Repair Regen. 2009 ; 17(4): 606-616),
- en diminuant aussi la formation de l'aquaporine 3, une protéine permettant l'apport d'eau dans l'épiderme pour hydrater la peau (Straseski J et al. Wound Repair Regen. 2009 ; 17(4): 606-616),
- en stimulant la production de molécules réactives de l'oxygène (ROS) qui altèrent les constituants et les cellules de la peau (Nys K et al. Free Radic Biol Med. 2012;52(6):1111-20)
- en inhibant la formation des kératines de différenciation comme la kératine 10 (Straseski J et al. Wound Repair Regen. 2009 ; 17(4): 606-616), et/ou
- en augmentant la présence des Matrix Metallo protéases ou MMP (Xia YP et al. J. Invest. Dermatol. 2001 ; 116 : 50-56) qui dégradent les constituants de la peau comme le collagène ce qui peut impacter la densité de la peau et sa fermeté et par conséquent l'ouverture des pores et la formation de rides et ridules.

On comprend donc l'intérêt, notamment pour des compositions de maquillage des matières kératiniques et notamment de la peau, ou des compositions pour peau grasse ou à tendance grasse, de disposer d'ingrédients susceptibles de préserver l'écosystème cutané des conséquences de l'hypoxie en assurant une au moins des actions protectrices suivantes :
- en préservant le microbiote et sa diversité en lui apportant les conditions cutanées qui le permettent, telles que le renforcement de la barrière cutanée et le contrôle de la production de sébum,
- en renforçant la cohésion de l'épiderme pour renforcer la résistance de la peau,
- en stimulant la formation de la barrière cutanée pour maintenir une hydratation suffisante dans la peau,
- en détoxifiant la peau des espèces réactives de l'oxygène,
- en inhibant la formation des MMPs, et/ou
- en stimulant la formation de l'Hypoxia inducing factor ou HIF qui assure une protection des cellules de la peau de l'hypoxie.

Il subsiste donc le besoin de développer de nouvelles compostions cosmétiques de maquillage des matières kératiniques, en particulier pour la peau, capables notamment de préserver la diversité du microbiote, de contrôler son développement et d'améliorer la qualité des matières kératiniques (peau, muqueuses, phanères) et notamment la peau, le relief et le grain de sa surface qui constitue une niche écologique pour ces microorganismes, en particulier de maintenir l'écosystème cutané et/ou maintenir l'équilibre du microbiote cutané, en particulier la diversité microbienne, de renforcer la barrière cutanée, d'améliorer la résistance de la peau aux stress, en particulier prévenir et/ou limiter les conséquences cutanées d'un environnement hypoxique, de réduire l'inflammation cutanée, de favoriser la desquamation et/ou le renouvellement épidermique, de diminuer la perte de fermeté impliquée dans le relâchement des pores et la formation des rides et les ridules, d'améliorer l'homogénéité du teint, et/ou de favoriser et/ou améliorer la tenue du maquillage et l'homogénéité de sa répartition à la surface de la peau.

Le Demandeur a mis en évidence que l'association d'un agent cosmétique constitué par des galactomannanes et des galactanes sulfatés réticulés, un prébiotique et un probiotique, permettait de répondre à ce besoin. En particulier, le Demandeur a pu montrer qu'une composition comprenant un agent cosmétique constitué par des galactomannanes obtenus à partir de *Caesalpinia spinosa* de masses molaires comprises entre 1 et 150kDa et de galactanes sulfatés réticulés obtenus à partir de *Kappaphycus alvarezii* de masses molaires comprises entre 1 et 150kDa, et un complexe pré-/pro-biotique comprenant un alpha-gluco-oligosaccharide, un extrait de racine de *Polymnia Sonchifolia* riche en fructo-oligosaccharide, un lysat de bactérie *Lactobacillus* et une maltodextrine, permettait de renforcer la formation de la barrière cutanée, d'améliorer la performance de la barrière cutanée, de favoriser le bon développement de l'épiderme et sa résistance, de maintenir l'intégrité des structures protéiques de la peau impliquées dans son maintien, dans l'effet astringent sur les pores et le comblement des reliefs de la peau, d'activer la capacité de la peau à résister à une situation d'hypoxie (manque d'oxygène), d'augmenter le contrôle exercé par la peau sur le microbiote, d'assurer l'homéostasie énergétique de la peau, d'améliorer et/ou renforcer la résistance de la peau aux stress, et diminuer l'inflammation.

### EXPOSE DE L'INVENTION

Un premier objet de l'invention concerne une composition cosmétique comprenant, dans un milieu physiologiquement acceptable au moins :
a) un agent cosmétique constitué par des galactomannanes de masses molaires comprises entre 5 et 630kDa, de préférence entre 5 et 120kDa, de préférence encore entre 8 et 80kDa, et des galactanes sulfatés réticulés de masses molaires comprises entre 7 et 3000kDa, de préférence entre 7 et 1100kDa, de préférence encore entre 8 et 200kDa, et
b) un prébiotique et un probiotique.

L'invention porte également sur un procédé cosmétique de soin et/ou de maquillage des matières kératiniques, en particulier de la peau, des lèvres et/ou des yeux, comprenant au moins l'application sur lesdites matières kératiniques d'une composition cosmétique de soin et/ou de maquillage, en particulier d'une composition de maquillage pour le teint, les lèvres ou les yeux, de préférence pour le teint.

Le procédé cosmétique est notamment destiné à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier favoriser ses capacités adaptatives aux conditions d'hypoxie et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, favoriser l'homéostasie énergétique de la peau, et/ou favoriser et/ou améliorer la tenue du maquillage et l'homogénéité de son dépôt.

L'invention concerne encore l'utilisation cosmétique non thérapeutique d'au moins un agent cosmétique constitué par des galactomannanes de masses molaires comprises entre 5 et 630kDa et des galactanes sulfatés réticulés de masses molaires comprises entre 7 et 3000kDa , un prébiotique et une fraction probiotique tels que définis selon l'invention, comme association destinée à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier favoriser ses capacités adaptatives aux conditions d'hypoxie et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, favoriser l'homéostasie énergétique de la peau, et/ou favoriser et/ou améliorer la tenue du maquillage et l'homogénéité de son dépôt.

### DEFINITIONS

Par 'matières kératiniques', on entend selon l'invention la peau, les muqueuses (en particulier les lèvres), et les phanères. Selon un mode particulier, la composition est destinée à une application topique sur la peau, les lèvres ou les phanères, de préférence la peau.

Par 'peau', on entend notamment la peau du visage et/ou du cou, en particulier la peau du visage, y compris les zones plus spécifiques des paupières (rentrant dans la catégorie de produits de maquillage des yeux) et du contour de l'œil.

Par 'microbiote cutané' ou 'microflore', on entend les micro-organismes (bactéries, levures, champignons, virus...) présents à la surface des matières kératiniques et notamment la peau.

Le terme 'microbiome cutané' désigne quant à lui l'ensemble de ces micro-organismes, leur génome et leurs interactions avec leur environnement.

Par 'écosystème cutané' autrement nommé 'biosphère cutanée', on entend selon l'invention le microbiote cutané et son hôte, la peau.

Par 'déséquilibre du microbiote cutané', on entend notamment un déséquilibre de la composition et/ou de la diversité du microbiote cutané.

Par 'maintenir l'écosystème cutané et/ou maintenir l'équilibre du microbiote cutané, en particulier la diversité microbienne', on entend selon l'invention la capacité des actifs ou d'une composition les contenant à préserver ou maintenir l'équilibre naturel de l'écosystème cutané, y compris en conditions de perturbations liées à des facteurs internes et/ou externes. Par 'maintenir l'équilibre du microbiote cutané', on entend notamment préserver ou maintenir la composition et/ou la diversité du microbiote, et contrôler son développement.

Par 'homogénéité du teint', on entend notamment un teint lumineux, homogène, on parle aussi d'éclat du teint, par opposition à 'altération du teint' qui désigne notamment un teint terne, inhomogène, brouillé, présentant des irrégularités de surface et/ou de couleur. Dans le cas des peaux grasses, l'altération du teint comprend en particulier un teint luisant et une perception d'inconfort et des manifestations ressenties comme des imperfections cutanées ou désordres esthétiques.

Par 'irrégularités de surface' autrement nommées 'imperfections cutanées', on entend notamment selon l'invention des irrégularités de relief (ex : les pores, les squames, les rides et ridules, un grain de peau imparfait) et/ou des irrégularités de couleur. Pour les lèvres, ces irrégularités de relief peuvent comprendre des gerçures ou crevasses.

Par 'prévenir et/ou limiter les conséquences cutanées d'un environnement hypoxique, on entend notamment selon l'invention maintenir la diversité du microbiote, et/ou prévenir et/ou améliorer les altérations de la peau liées à une condition transitoire d'hypoxie, telles qu'une diminution de la cohésion cellulaire, une diminution de l'apport en eau dans l'épiderme, une augmentation de la production des molécules réactives de l'oxygène, une inhibition de la formation des kératines de différenciation et/ou une augmentation de la dégradation du collagène.

Par 'favoriser les capacités adaptatives de la peau aux conditions d'hypoxie', on entend selon l'invention l'effet de la composition cosmétique de l'invention sur la capacité de la peau à résister à une situation d'hypoxie (manque d'oxygène) en stimulant l'expression de gènes induits lorsque la teneur en oxygène de la peau est faible et en permettant au métabolisme de s'adapter à ce nouvel environnement.

Par 'favoriser l'homéostasie énergétique de la peau', on entend selon l'invention la capacité de la composition cosmétique à assurer l'homéostasie énergétique de la peau en contribuant à la formation d'ATP, molécule de stockage de l'énergie cellulaire utilisée pour assurer l'ensemble du métabolisme des cellules cutanées.

Par 'peau grasse ou à tendance grasse', on entend notamment une peau grasse caractérisée par une présence excessive en sa surface de sébum, produit de sécrétion des glandes sébacées. Cette production de sébum est assurée plus particulièrement par les sébocytes au travers d'un processus de différenciation cellulaire et de synthèse ou accumulation de lipides appelé lipogenèse. On parle également de peau hyperséborrhéique.

Une peau grasse est souvent associée à un défaut de desquamation, un terrain inflammatoire et/ou une oxydation des lipides, pouvant provoquer un teint luisant, un grain de peau imparfait, une perception d'inconfort et manifestations ressenties comme des imperfections cutanées ou désordres esthétiques ; une telle peau présente également une moins bonne tenue du maquillage au cours de la journée et particulièrement l'après-midi lorsque la production sébacée est maximale.

Outre son aspect disgracieux, une peau grasse peut être un terrain favorable à la prolifération de micro-organismes et participer ainsi à la survenue de désordres cutanés (ex : lésions d'acné). On parlera alors de peau grasse ou hyperséborrhéique à tendance acnéique.

Par 'désordres esthétiques cutanés ou désordres non pathologiques associés aux peaux grasses ou à tendance grasse', on entend notamment des désordres dus à une hyper-séborrhée, un défaut de desquamation, un environnement hypoxique et/ou à un déséquilibre du microbiote. En particulier, les désordres esthétiques cutanés associés sont des désordres non pathologiques sélectionnés parmi une peau présentant un déséquilibre du microbiote cutané, un défaut de desquamation, une oxydation des lipides, une altération du teint en particulier un teint luisant, une perception d'inconfort, des irrégularités de surface, en particulier des orifices folliculaires ou des pores dilatés, un grain de peau imparfait, et/ou une peau présentant une moins bonne tenue du maquillage.

### DESCRIPTION DETAILLEE DE L'INVENTION

L'invention concerne donc notamment une composition cosmétique comprenant, dans un milieu physiologiquement acceptable au moins :
a) un agent cosmétique constitué par des galactomannanes de masses molaires comprises entre 5 et 630kDa, de préférence entre 5 et 120kDa, de préférence encore entre 8 et 80kDa, et des galactanes sulfatés réticulés de masses molaires comprises entre 7 et 3000kDa, de préférence entre 7 et 1100kDa, de préférence encore entre 8 et 200kDa, et
b) un prébiotique et un probiotique.

### Galactomannanes et Galactanes sulfatés réticulés

L'agent cosmétique au sens de l'invention est constitué de biopolymères, c'est-à-dire de polymères issus de matières végétales, qui sont obtenus par synthèse chimique.

Ces biopolymères ont un effet filmogène, c'est-dire un effet pouvant créer à la surface de la peau un film non perceptible à l'œil nu, afin de protéger la peau des agressions externes telle que la pollution et les allergènes.

De tels biopolymères sont décrits notamment dans les demandes de brevets WO2017/19792 et WO2017/129780 de la Société Industrielle Limousine d'Application Biologique.

L'agent cosmétique ou dermocosmétique utilisé selon l'invention est constitué par :
- des galactomannanes de masses molaires comprises entre 5 et 630kDa, et
- des galactanes sulfatés réticulés de masses molaires comprises entre 7 et 3000kDa.

Par 'masse molaire moyenne' d'un mélange de molécules, on entend la moyenne des masses molaires pondérales de chaque molécule du mélange.

Par 'réticulé', on entend au sens de l'invention un biopolymère dans lequel a été formé un réseau tridimensionnel au moyen de la formation de liaisons chimiques ou physiques entre les molécules du biopolymère.

Préférentiellement, l'agent cosmétique ou dermocosmétique est constitué par :
- des galactomannanes de masse molaire moyenne comprise entre 5 et 120kDa, et des galactanes sulfatés réticulés de masse molaire moyenne comprise entre 7 et 1100kDa.

Encore plus préférentiellement l'agent cosmétique ou dermocosmétique est constitué par :
- des galactomannanes de masse molaire moyenne comprise entre 8 et 80kDa, et des galactanes sulfatés réticulés de masse molaire moyenne comprise entre 8 et 200kDa.

Les galactomannanes peuvent être obtenus notamment à partir d'hydrolyse de galactomannanes natifs de Tara (*Caesalpinia spinosa),* de guar (*Cyamopsis tetragonoloba*)*,* de caroube (*Ceratonia siliqua*), mais aussi de séné (*Cassia angustifolia*), de cassier (*Cassia fistula*), de cassia (*Cassia obtusifolia* ou *Cassia tora*), de caroube chinois (*Gleditsia sinensis),* de févier (*Gieditsia triacanthos*), de sophora (*Sophora japonica*) et/ou de fenugrec (*Trigonella foenum-graecum*), de préférence de *Caesalpinia spinosa* .

Les galactanes sulfatés réticulés peuvent être obtenus par hydrolyse de galactanes sulfatés natifs de carraghénanes (de *Kappaphycus alvarezii,* de *Kappaphycus striatum, d'Eucheuma cottonii, d'Eucheuma spinosum,* de *Chondrus crispus,* de *Gigartina skottsbergii,* de *Sacrothalia crsipata*), ou de *Fucellaria fastigiata,* d'Agar *(Gelidium sesquipedale)* ou des algues (*Polysiphonia lanosa* ou *Codium fragile),* de préférence de *Kappaphycus alvarezii.* Selon un mode particulier de l'invention, l'agent cosmétique est constitué de galactomannanes obtenus par hydrolyse de galactomannanes natifs de Tara (Caesalpinia spinosa), de guar (Cyamopsis tetragonoloba), de caroube (Ceratonia siliqua), mais aussi de séné (Cassia angustifolia), de cassier (Cassia fistula), de cassia (Cassia obtusifolia ou Cassia tora), de caroube chinois (Gleditsia sinensis), de févier (Gieditsia triacanthos), de sophora (Sophora japonica) et/ou de fenugrec (Trigonella foenum-graecum), de préférence de Caesalpinia spinosa ; et de galactanes sulfatés réticulés obtenus par hydrolyse de galactanes sulfatés natifs de carraghénanes (de Kappaphycus alvarezii, de Kappaphycus striatum, d'Eucheuma cottonii, d'Eucheuma spinosum, de Chondrus crispus, de Gigartina skottsbergii, de Sacrothalia crsipata), ou de Fucellaria fastigiata, d'Agar (Gelidium sesquipedale) ou des algues (Polysiphonia lanosa ou Codium fragile), de préférence de Kappaphycus alvarezii.

Les galactomannanes et galactanes sulfatés réticulés sont obtenus selon les étapes suivantes :
- Solubilisation de poudre de galactomannanes natifs ou respectivement des galactanes sulfatés natifs dans l'eau,
- Hydrolyse ménagée pat voie chimique ou enzymatique ;
- Séparation des phases solubles ou insolubles, afin d'éliminer la phase insoluble ;
- Sélection par filtration(s) membranaire(s) des galactomannanes de masse molaire définie.

Les galactanes sulfatés réticulés sont aussi réticulés par un agent réticulant, préférentiellement un agent réticulant de nature ionique.

Les galactomannanes et galactanes sulfatés réticulés ainsi obtenus sont mélangés pour constituer un agent cosmétique.

Selon un mode particulier et préféré, l'agent cosmétique a) selon l'invention est constitué de 60 à 90% de galactomannanes et 10 à 40% de galactanes sulfatés réticulés.

Selon un mode particulier et préféré, l'agent cosmétique est constitué de galactomannanes obtenus à partir de *Caesalpinia spinosa* de masses molaires comprises entre 1 et 150kDa, et de galactanes sulfatés réticulés obtenus à partir de *Kappaphycus alvarezii* de masses molaires comprises notamment entre 1 et 150kDa.

Selon un mode particulier de l'invention, l'agent cosmétique a) est présent dans la composition en une teneur allant de 0,01% à 2% en poids de matière active par rapport au poids total de la composition, préférentiellement 0,1% à 0,1%, et préférentiellement encore de 0,4% à 0,8% en poids de matière active par rapport au poids total de la composition.

Par 'matière active', on entend les composés actifs de l'agent cosmétique auxquels est attribuée l'efficacité. On parle également de matière sèche pour les extraits végétaux.

De tels biopolymères sont décrits dans les demandes de brevets WO2017/19792 et WO2017/129780 de la Société Industrielle Limousine d'Application Biologique, en particulier l'exemple 1 de la demande WO2017/129780. L'utilisation de ces biopolymères permet d'améliorer l'effet barrière cutané par un effet protecteur de la peau contre la pénétration de molécules toxiques, telles que les polluants, les allergènes, les métaux lourds. L'effet de film après application su la peau permet également d'améliorer l'apparence globale du visage et favoriser la tenue des pigments et du maquillage.

Selon un mode particulier et préféré, on utilisera comme agent cosmétique constitué par des galactomannanes et des galactanes sulftates réticulé un produit commercialisé par la société SILAB sous la dénomination commerciale FILMEXEL^{®}, de nom INCI 'CAESALPINIA SPINOSA EXTRACT and KAPPAPHYCUS ALVAREZII EXTRACT and WATER', sous forme de poudre et de composition CAESALPINIA SPINOSA EXTRACT 76%, KAPPAPHYCUS ALVAREZII EXTRACT 19%, and WATER 5% (95% de matière active ou matière sèche).

### Complexe prébiotique/probiotique (synbiotique)

On entend par « prébiotique » des constituants alimentaires non vivants qui ont la propriété de moduler la croissance et l'activité des souches probiotiques (lactobacilles, bifidobactéries...) et des autres souches bactériennes de l'intestin bénéfiques à la santé humaine. Les prébiotiques sont généralement des monosaccharides, disaccharides ou oligosaccharides, qui peuvent se retrouver naturellement dans les fruits, les légumes, et le miel et sont extractibles. D'autres sont produits industriellement par hydrolyse des polysaccharides (par exemple des fructo-oligosides ou oligofructoses, abrégé en FOS pour Fructo-oligosaccharides) ou synthétisés en soumettant des disaccharides tels que le lactose à l'action d'enzymes comme les lactases avec des activités transférases pour produire des trans-galacto-oligosaccharides (TOS) ou par une réaction chimique d'isomérisation qui donne lelactulose. Actuellement, les trans-galactooligosaccharides (GOS) et les fructanes de type inuline sont ceux dont les effets prébiotiques sont reconnus.

On entend par « probiotique ou dérivé » des micro-organismes vivants ou inactivés qui, lorsqu'ils sont ingérés en quantité suffisante, exercent des effets positifs sur la santé au-delà des effets nutritionnels traditionnels.

Les probiotiques peuvent être des bactéries ou des levures.

Le probiotique ou dérivé peut être choisi parmi une souche d'un ou plusieurs des suivants: *Lactobacillus,* souches et dérivés de *Clostridia,* souches et dérivés de *Bifidobacterium,* souches et dérivés de *Saccharomyces,* souches et dérivés de *Lactococcus,* souches et dérivés de *Pedicoccus,* souches et dérivés de Enterococcus, souches et dérivés *d'Escherichia,* souches et dérivés d'*Alcaligenes,* souches et dérivés de *Corynebacterium,* souches et dérivés de *Bacillus,* et souches et dérivés de *Propionibacterium.*

On peut citer notamment les bactéries des genres *Lactobacillus* et *Bifidobacterium.* Les souches identifiées comme probiotiques s'appliquent principalement à la sphère intestinale, il s'agit notamment de *Lactobacillus acidophilus, L. casei, L. gasseri, L. paracasei, L. Rhamnosus* et *Bifidobacteria animal, B. breve, B. longum.*

Comme levures, on peut citer notamment *Saccharomyces cerevisiae var. boulardii* qui se trouve de façon naturelle dans le litchi.

On entend par 'synbiotiques' des associations de probiotiques et de prébiotiques.

Selon un mode particulier, le prébiotique est choisi dans le groupe constitué par l'inuline et l'alphaglucane oligosaccharide.

Selon un mode particulier, le probiotique est choisie dans le groupe constitué par des lysats de probiotiques. De préférence, le lysat de probiotique est un lysat du genre *Bacillus.*

Selon un mode particulier et préféré, la composition cosmétique de l'invention comprend comme complexe prébiotique/probiotique Ecoskin^{®} commercialisé par la société SOLABIA, qui est un mélange d'oligosaccharide d'alpha-glucane, de jus de racine de sonchifolia de polymnia, de maltodextrine, et de bactéries de *Lactobacillus sp,* de nom INCI : ALPHA-GLUCAN OLIGOSACCHARIDE and POLYMNIA SONCHIFOLIA ROOT JUICE and MALTODEXTRIN and LACTOBACILLUS

La composition de Ecoskin^{®} est : ALPHA-GLUCAN OLIGOSACCHARIDE 70%, POLYMNIA SONCHIFOLIA ROOT JUICE 19%, MALTODEXTRIN 10%, LACTOBACILLUS 1% (correspondant à 90% de matière active).

C'est un complexe pré/probiotique, séché par pulvérisation sur la maltodextrine, fait de: α-gluco-oligosaccharides (GOS) obtenu par synthèse enzymatique à partir de substrats végétaux (maltose de maïs, saccharose de betterave), 100% de jus végétaux purs riches en β-fructooiigosaccharides (FOS) obtenus par pressage à froid de tubercules de *Polymnia sonchifolia,* et d'une bactérie probiotique de *Lactobacillus (L. casei, L. acidophilus),* inactivée par tyndallisation et lyophilisée.

La portion prébiotique provient des α-gluco-oligosaccharides (GOS) et des β-fructooligosaccharides de l'extrait pressé à froid de tubercules de *Polymnia sonchifolia* ; ces prébiotiques stimulent l'écoflore de la peau.

Les probiotiques sont les bactéries *Lactobacillus* inactivées qui stimulent les β-défensines qui sont impliquées dans le système de défense de la peau. Les souches de bactéries Lactobacillus (*Lactobacillus casei* et *Lactobacillus acidophilus*) qui sont utilisées dans Ecoskin^{®} sont auparavant lyophilisées et tyndallisées, ce qui signifie que leur système de reproduction est inactivé par la chaleur empêchant leur développement dans les préparations cosmétiques qui les contiennent.

Selon un mode particulier, le complexe prébiotique/probiotique est présent dans la composition en une teneur allant de 0,05% à 3% en poids de matière active par rapport au poids total de la composition, préférentiellement de 0,1 à 1% en poids de matière active par rapport au poids total de la composition.

Selon un mode particulier, la composition cosmétique peut comprendre en outre au moins un hydrolysat de fèves de *Theobroma cacao L.*

### Hydrolysat de Theobroma cacao L

L'hydrolysat de *Theobroma cacao L.* selon l'invention est un hydrolysat peptidique et osidique des fèves de *Theobroma cacao L* comprenant majoritairement des peptides et des osides. On entend par "majoritairement des peptides et des osides" une quantité supérieure à 50%, préférentiellement supérieure à 60%, préférentiellement encore supérieure à 70% et pouvant atteindre environ 90% (poids/poids) de matière sèche en peptides et en oses, préférentiellement 90% du poids de la matière sèche.

On entend par "hydrolysat peptidique et osidique" un hydrolysat comprenant majoritairement ou essentiellement des peptides et des oses (mono et oligosaccharides). Les protéines et les polysaccharides naturellement présents dans les fèves ont été hydrolysés en peptides, en oligosaccharides et en monosaccharides, avantageusement l'hydrolyse est une hydrolyse enzymatique.

Cet hydrolysat peut être obtenu comme suit :
a) on met en dispersion en phase aqueuse des fèves broyées de *Theobroma cacao L* ;
b) on effectue un traitement enzymatique de la dispersion aqueuse obtenue à l'étape a) ;
c) on effectue la récupération de l'hydrolysat enzymatique par séparation solide / liquide,
d) on purifie l'hydrolysat par ultrafiltration et nanofiltration, puis éventuellement ; e) on effectue une lyophilisation de l'hydrolysat obtenu à l'étape d).

L'hydrolysat utilisé selon l'invention est obtenu à partir de fèves de *Theobroma cacao L,* comme matière de départ, qui peuvent comprendre indifféremment la fève seule ou la fève et son enveloppe, préférentiellement on utilisera les fèves comprenant la fève et son enveloppe. L'hydrolysat de cacao non lyophilisé utilisé selon l'invention comprend de 20 à 70% de peptides et de 5 à 40% d'osides. L'hydrolysat de cacao lyophilisé sans support de séchage selon l'invention comprend plus de 90 pourcent de matière sèche comprenant de 20 à 70% de peptides et de 5 à 40% d'osides.

Dans un autre mode de réalisation très préféré selon l'invention, les peptides et osides présents dans l'hydrolysat utilisé selon l'invention présentent un poids moléculaire compris entre 200 Da et 10 kDa.

Ainsi, selon un mode particulier et préféré, l'hydrolysat de fèves de Theobroma cacao L est un hydrolysat peptidique et osidique de fèves de Theobroma cacao L, comprenant notamment peptides et osides présentant un poids moléculaire compris entre 200 Da et 10 kDa. L'hydrolysat ainsi obtenu peut ensuite être encore dilué dans de l'eau ou dans tout mélange de solvants contenant de l'eau. Ainsi, l'hydrolysat de cacao selon l'invention peut avantageusement être dilué dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. Pour préparer la composition, l'hydrolysat de cacao selon l'invention peut se trouver sous forme liquide ou lyophylisée. Selon un mode particulier, il est sous une forme liquide.

Selon un mode particulier et préféré, on utilisera comme hydrolysat de Theobroma cacao L. un produit commercialisé par la société ASHLAN sous la dénomination commerciale BLUMILIGHT^{™}, de nom INCI 'BUTYLENE GLYCOL and WATER and THEOBROMA CACAO (COCOA) SEED EXTRACT', sous forme d'un liquide et de composition BUTYLENE GLYCOL 55%, WATER 43.75%,THEOBROMA CACAO (COCOA) SEED EXTRACT 1.25% (1.25% de matière active ou matière sèche).

Selon un mode particulier, l'hydrolysat de fèves de Theobroma cacao L. est présent dans la composition en une teneur allant de 0,0005 à 0,025% en poids de matière active par rapport au poids total de la composition, préférentiellement de 0,001 à 0,01% en poids de matière active par rapport au poids total de la composition.

### Galénique

Les compositions selon l'invention sont destinées plus particulièrement à une application topique sur les matières kératiniques, en particulier sur la peau.

Les compositions de l'invention comprennent un milieu cosmétiquement acceptable, c'est-à-dire compatible avec la peau et les phanères. Les compositions peuvent avoir toutes les formes cosmétiques, et notamment être sous forme de crèmes, émulsions huile-dans-eau, ou eau-dans-huile ou émulsions multiples, solutions, suspensions, gels, laits, lotions, sérums, sticks ou encore de poudres, et adaptées à une application sur la peau, les lèvres et/ou les phanères.

Selon un mode particulier, la composition de l'invention est destinée à une application topique sur la peau, les lèvres ou les yeux et se présente sous la forme d'un produit de soin et/ou d'un produit de maquillage pour la peau, les lèvres et ou les yeux, en particulier de la pour le teint.

On parlera indifféremment de maquillage de la peau ou maquillage pour le teint.

Par 'maquillage des yeux', on entend notamment des liners, ou des fards à paupières.

Selon un mode particulier et préféré, la composition de l'invention se présente sous la forme d'un fond de teint, ou d'une base de teint, de préférence d'un fond de teint.

Selon un mode particulier et préféré, la composition de l'invention se présente sous la forme d'une poudre de teint, d'un fond de teint, ou d'une base de teint, de préférence d'un fond de teint.

La composition cosmétique de l'invention est avantageusement sous la forme d'une émulsion huile-dans-eau, une émulsion eau-dans-huile, une émulsion multiple, un gel aqueux.

La composition est de préférence sous la forme d'une émulsion contenant une phase aqueuse et une phase huileuse.

La phase aqueuse représente généralement de 1 à 99% en poids, par rapport au poids total de ladite composition.

La composition de l'invention comprend généralement, outre la phase aqueuse, également une phase huileuse.

Une phase huileuse selon l'invention peut comprendre des huiles hydrocarbonées, siliconées, fluorées ou non, et leurs mélanges. Ces huiles peuvent être volatiles ou non volatiles, végétale, minérale ou synthétique.

Avantageusement, on utilisera des huiles hydrocarbonées.

Comme huiles volatiles hydrocarbonées, on peut citer notamment les alcanes ramifiés en C8-C16, les esters ramifiés en C8-C16 et leurs mélanges.

Comme huiles non volatiles hydrocarbonées, on peut citer notamment les huiles hydrocarbonées, les huiles hydrocarbonées d'origine végétale, les éthers de synthèse en C10-C40, les esters de synthèse en C10-C40, les alcools gras en C12-C26, les acides gras supérieurs en C12-C22, et leurs mélanges.

Les huiles pourront être présentes dans la composition de l'invention en une teneur allant de 1 à 95 % en poids par rapport au poids total de la composition.

La composition de l'invention peut également comprendre tout additif usuellement utilisé en cosmétique tels que des filtres UV, des antioxydants, des tensio-actifs, des gélifiants, des charges, matières colorantes, des conservateurs, des polymères filmogènes, des parfums, des agents actifs cosmétiques, comme par exemple des émollients, des hydratants, des vitamines, des agents anti-âge, des agents éclaircissants, et leurs mélanges.

Selon un mode particulier de l'invention, la composition cosmétique comprend au moins une ou plusieurs matières colorantes.

Les matières colorantes peuvent être choisies parmi les matières colorantes hydrosolubles ou non, liposolubles ou non, organiques ou inorganiques, les matériaux à effet optique, et leurs mélanges.

On entend par matière colorante au sens de la présente invention, un composé susceptible de produire un effet optique coloré lorsqu'il est formulé en quantité suffisante dans un milieu cosmétique approprié.

Selon un mode particulier, la ou les matières colorantes sont notamment choisies parmi des pigments minéraux et/ou organiques, des pigments composites (à base de matériaux minéraux et/ou organiques), des colorants, des nacres ou pigments nacrés, et leurs mélanges.

Par pigments, il faut comprendre des particules blanches ou colorées, inorganiques (minérales) ou organiques, insolubles dans la phase organique liquide, destinées à colorer et/ou opacifier la composition et/ou le dépôt réalisé avec la composition.

Parmi les pigments minéraux, on peut citer, à titre d'exemples le dioxyde de titane (rutile ou anatase), éventuellement traité en surface ; les oxydes de fer noir, jaune, rouge et brun ; le violet de manganèse ; le bleu outremer l'oxyde de chrome l'oxyde de chrome hydraté et le bleu ferrique.

Pour les compositions destinées aux lèvres, on peut citer à titre d'exemples le dioxyde de titane; les oxydes de fer noir, jaune, rouge et brun et le violet de manganèse.

Parmi les pigments organiques, on peut citer, par exemple, les pigments D & C red n° 19; D & C red n° 9; D & C Red n° 22 ; D & C Red n° 21 ; D & C Red n° 28 ; D & C Yellow n° 6 ; D & C orange n° 4 ; D & C orange n° 5 ; D & C Red n° 27; D & C red n° 13; D & C Red n° 7 ; D & C Red n° 6 ; D & C Yellow n° 5; D & C Red n° 36 ;; D & C Red n° 33 ; D & C orange n° 10; D & C yellow n° 6 ; ; D & C Red n° 30 ; D &C red n° 3 ; D &C Blue 1 ; le noir de carbone et les laques à base de carmin de cochenille.

Parmi les colorants hydrosolubles, on pourra citer Yellow 5, Yellow 6, Blue 1, Green 5, Green 3, Green 6, Orange 4, Red 4, Red 21,Red 22, Red 27, Red 28, Red 33, Red 40, le carminé de cochenille (CI 15850, CI 75470).

Les colorants liposolubles sont par exemple le rouge Soudan, le D&C Red 17, le D&C Green 6, le bêta-carotène, l'huile de soja, le brun Soudan, le D&C Yellow 11, le D&C Violet 2, le D&C Orange 5, le jaune quinoléine, le rocou.

Les nacres ou pigments nacrés peuvent être choisis notamment parmi les pigments nacrés blancs, tels que le mica recouvert d'oxyde de titane, l'oxychlorure de bismuth ; et les pigments nacrés colorés, tels que le mica titane avec des oxydes de fer, le mica titane avec du bleu ferrique ou de l'oxyde de chrome, le mica titane avec un pigment organique du type précité, ainsi que les pigments à base d'oxychlorure de bismuth. On pourra citer les gammes de nacres de référence commerciale Reflecks^{®}, Ronastar^{®}, Timiron^{®} et Syncristal^{®}.

En particulier, la ou les matières colorantes sont présentes dans la composition en une teneur allant de 2% à 30% en poids, de préférence de 4% à 15% en poids par rapport au poids total de la composition.

Selon un mode particulier, la composition de l'invention comprend des pigments, en particulier des pigments minéraux en une teneur allant de 5 à 25%, notamment de 10 à 20% en poids par rapport au poids total de la composition.

Les charges sont choisies notamment parmi les silices, les micas, d'origine naturelle ou synthétique, le kaolin, les oxydes de zinc et de titane ; le carbonate de calcium, le carbonate et l'hydrocarbonate de magnésium ; le stéarate de zinc, de magnésium ou de lithium, le laurate de zinc, le myristate de magnésium ; les poudres de polymères synthétiques, tels que le polyéthylène, les polyesters, les polyamides (par exemple le nylon) ; des poudres d'acides polyacrylique ou polyméthacrylique, des poudres de résine de silicone ; les poudres minérales telles que la silice sphérique ; les dioxydes de titane sphériques; les billes de verre et de céramique ; des poudres de matériaux organiques d'origine naturelle comme les amidons de maïs, de blé, de riz, réticulées ou non, et leurs mélanges.

Selon un mode particulier, la composition de l'invention comprend des pigments, en particulier des pigments minéraux en une teneur allant de 5 à 25%, notamment de 10 à 20% en poids par rapport au poids total de la composition.

Selon un mode particulier de l'invention, la composition cosmétique ne contient pas d'autres actifs cosmétiques que ceux objets de l'invention.

Selon un autre mode particulier de l'invention, la composition contient en outre d'autres actifs cosmétiques et/ou dermatologiques. On peut citer notamment les actifs visant à stimuler la biodiversité et/ou l'homéostasie du microbiote cutané, le renouvellement cellulaire, la régénération ou revitalisation du teint (éclat), la réduction de la production de sébum, la protection vis-à-vis d'agressions externes.

On peut citer notamment les actifs suivants visant à stimuler :
- la biodiversité et l'homéostasie du microbiote de la peau, comme le fait l'actif Actibiome^{©} (Water and seawater and glycerin and Laminaria digitata extract and Chlorella vulgaris extract and saccharide isomerate and phenoxyethanol and ethylhexylglycerin), en rééquilibrant le pH de la peau précédemment diminué par une période de stress ;
- le renouvellement cellulaire, avec l'aide d'actifs à effet « peeling » seuls ou en combinaison et à pH acide :
   ▪ par voie chimique, comme les Acides Alpha Hydroxylés AHA (acide glycolique, lactique, citrique...), Acides Beta Hydroxylés BHA (acide salicylique), Acides Poly Hydroxylés PHA (gluconolactone) ;
   ▪ par voie biologique, comme Exfolactive C EL PX^{©} (Opuntia coccinellifera), DERMOCH DP^{©} (Chlorella vulgaris), ALGOWHITE^{©} (Ascophyllum nodosum)
   ▪ par voie enzymatique, comme MELACLEAR^{©} (Gluconic acid, Sutilains) ;
- la régénération ou revitalisation du teint (éclat), à l'aide d'actifs :
   ▪ lissants comme le Retinol (vitamine A-like)
   ▪ éclaircissants par action sur la mélanogénèse (dérivé de vitamine C)
   ▪ énergisants tels que des nutriments et/ou anti-oxydants vitaminiques C et E comme SEPIVITAL^{©} (potassium ascorbyl tocopheryl phosphate)
- la réduction de la production de sébum, à l'aide d'agents sébo-capteurs (Argiles) ou actifs sébo-régulateurs comme le gluconate de Zinc (Mineralis GU / Zn) et l'extrait lipophyle d'avocat (5 alpha avocuta),
- la protection vis-à-vis d'agressions externes : pollution, lumière bleue, UV à l'aide de filtres chimiques ou minéraux et/ou particules réfléchissantes diffusantes, et leurs mélanges.

L'invention porte également sur un procédé cosmétique de soin et/ou de maquillage des matières kératiniques, en particulier de la peau, des lèvres et/ou des yeux, comprenant au moins l'application sur lesdites matières kératiniques d'une composition cosmétique de soin et/ou de maquillage telle que définie selon l'invention, en particulier d'une composition de maquillage pour le teint, les lèvres ou les yeux, de préférence pour le teint.

Le procédé cosmétique est notamment destiné à à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier favoriser ses capacités adaptatives aux conditions d'hypoxie et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, favoriser l'homéostasie énergétique de la peau, et/ou favoriser et/ou améliorer la tenue du maquillage et l'homogénéité de son dépôt.

L'invention concerne encore l'utilisation cosmétique non thérapeutique d'au moins un agent cosmétique constitué par des galactomannanes de masses molaires comprises entre 5 et 630kDa et des galactanes sulfatés réticulés de masses molaires comprises entre 7 et 3000kDa , un prébiotique et une fraction probiotique tels que définis selon l'invention, comme association destinée à à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier favoriser ses capacités adaptatives aux conditions d'hypoxie et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, favoriser l'homéostasie énergétique de la peau, et/ou favoriser et/ou améliorer la tenue du maquillage et l'homogénéité de son dépôt.

L'invention va être illustrée par les exemples non limitatifs suivants. Les pourcentages sont exprimés en pourcentage en poids par rapport au poids total de la composition sauf indication contraire.

### EXEMPLES

### Exemple 1 : Effet de l'association d'un extrait de Caesalpinia spinosa et d'un extrait de Kappaphycus alvarezii (Filmexel^{®} ) en combinaison avec Ecoskin^{®} sur les kératinocytes

Une étude sur les effets transcriptionnels de ces actifs, utilisés seuls ou en association, a été réalisée à l'aide de la technologie TLDA (TaqMan Low Density Array). Grâce à cette technologie, la modulation de l'expression de gènes codant pour des protéines spécifiques est étudiée en réponse à un traitement de Kératinocytes Humains Normaux, ce qui permet une cartographie de l'expression génique des actifs seuls ou en association.

Cette approche permet, notamment, de mettre en évidence les effets bénéfiques des actifs ou de leur combinaison dans les domaines couverts par le profil : formation de l'épiderme et desquamation, matrice extracellulaire, détoxification et anti-oxydation, inflammation et immunité innée.

### Matériel et méthodes

Cellules utilisées : Kératinocytes normaux d'épiderme humain
Milieu de culture : EpiLife^{®} complété par du HKGS (Human-Keratinocyte-Growth-Supplement)
Origine cutanée des cellules : prélèvement abdominal, femme, 47 ans
Modèle d'étude : Technologie TLDA (TaqMan Low Density Array)
Traitement des cellules par les composés : 24h
Composés testés :
   - ECOSKIN^{®} (ALPHA-GLUCAN OLIGOSACCHARIDE and POLYMNIA SONCHIFOLIA ROOT JUICE and MALTODEXTRIN and LACTOBACILLUS) à 0.25%
   - FILMEXEL^{®} (CAESALPINIA SPINOSA EXTRACT and KAPPAPHYCUS ALVAREZII EXTRACT and WATER) à 0.25%
   - ECOSKIN^{®} à 0.25% + FILMEXEL^{®} à 0.25%

### Résultats

Le tableau 1 ci-dessous retranscrit les variations d'activités pour les gènes étudiés.

Les conditions expérimentales retenues sont les suivantes :
- Chaque essai est réalisé en triplicate (3 non traités et 3 traités dans les mêmes conditions) ;
- Le test-F de Fischer est d'abord appliqué en comparant les deux matrices de données. Lorsque la valeur est supérieure à α = 0,05 alors la variance du test t de Student est de 2, lorsque le test-F de Fischer est inférieur à α = 0,05, alors la variance sera égale à 3.
- Les variations transcriptionnelles retenues (significatives) seront celles qui correspondent à un test t de Student inférieur ou égal à α (ou p) = 0,05. En gris foncé les stimulations (voir ci-dessous) le fond blanc indiquant les variations non significatives c'est-à-dire celles correspondant à un test t de Student supérieur à alpha (ou p) = 0,05.

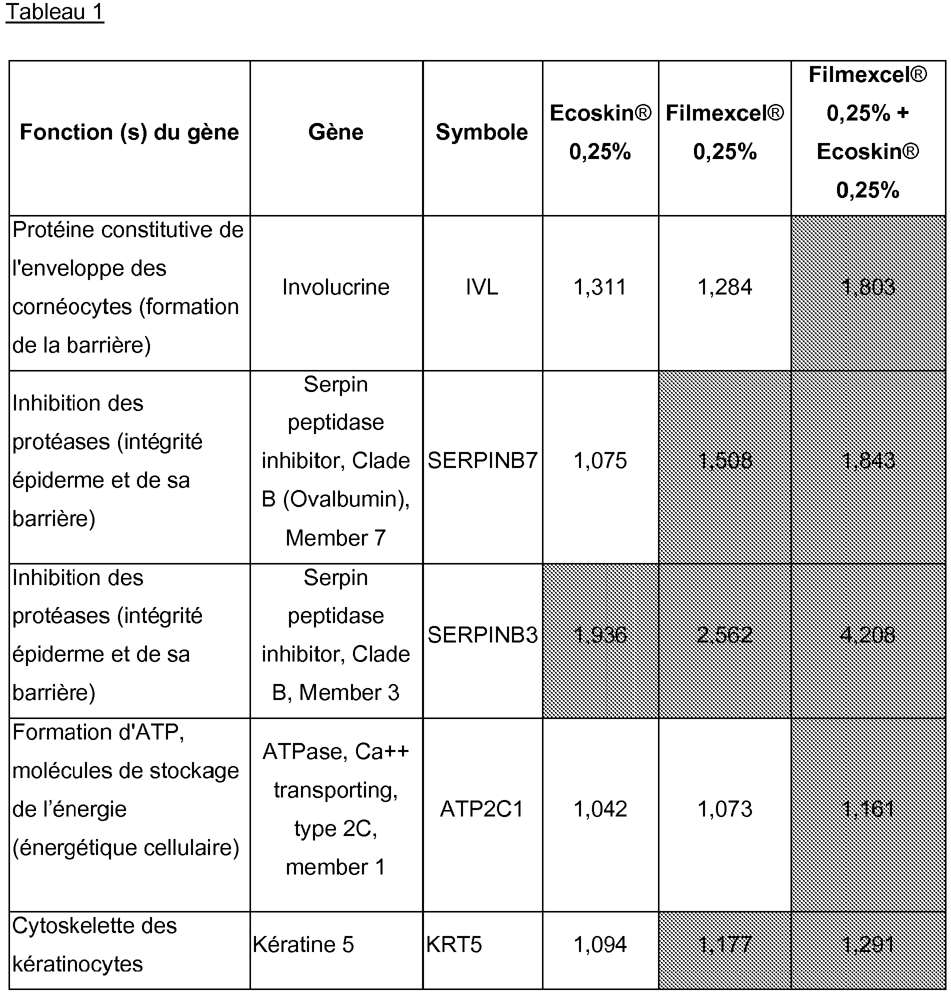

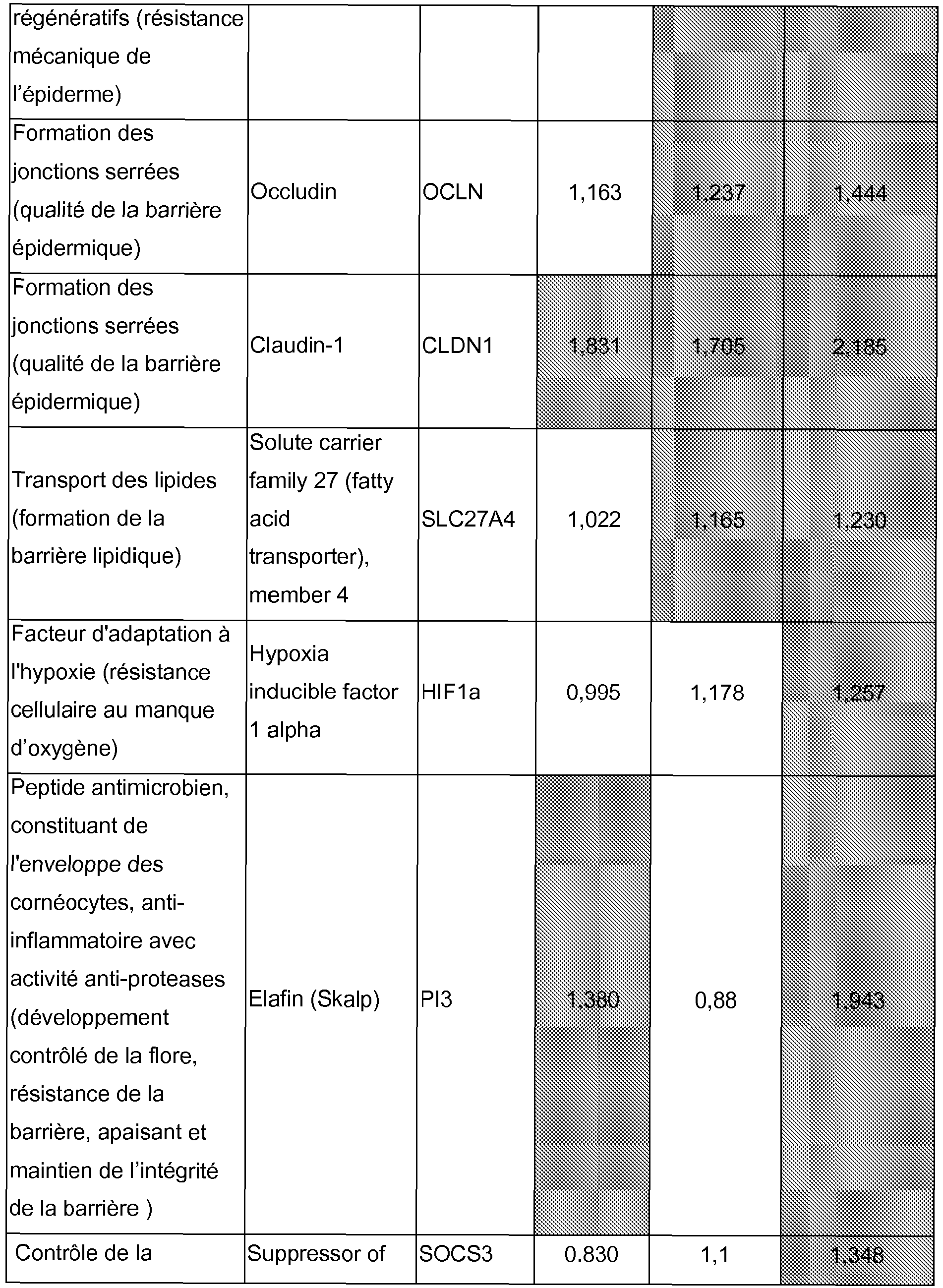

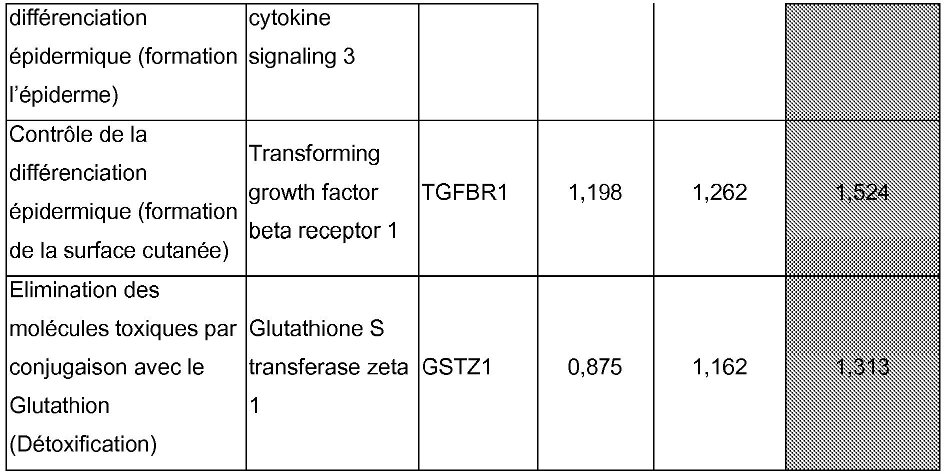

Ces données mettent en évidence, pour les associations testées, un effet synergique permettant :
- de renforcer la formation de la barrière cutanée en particulier la formation des corneocytes (enveloppe cellulaire) en stimulant l'activité transcriptionnelle des gènes IVL et PI3
- d'améliorer la performance de la barrière cutanée en stimulant l'activité transcriptionnelle des gènes CLDN1 et OCLN, impliqués dans la formation des jonctions serrées intercellulaires qui limitent le passage de l'eau intercellulaire (perte insensible en eau) et maintiennent les lipides épidermiques à la surface cutanée, et en stimulant l'activité transcriptionnelle du gène SLC27A4 impliqué dans le transport des lipides épidermiques
- de favoriser le bon développement de l'épiderme et sa résistance en particulier en stimulant l'activité transcriptionnelle des gènes SOCS3, TGFBR1, KRT5,
- de maintenir l'intégrité des structures protéiques de la peau impliquées dans son maintien, dans l'effet astringent sur les pores et le comblement des reliefs de la peau, en empêchant la dégradation de ces structures protéiques par l'activation des gènes des inhibiteurs de protéases comme la SERPINB3, SERPINB7 et PI3
- d'activer la capacité de la peau à résister à une situation d'hypoxie (manque d'oxygène) stimulant l'activité transcriptionnelle du gène HIF1a induit lorsque la teneur en oxygène de la peau est faible et permet au métabolisme de s'adapter à ce nouvel environnement tissulaire,
- d'augmenter le contrôle exercé par la peau sur le microbiote en stimulant l'activité transcriptionnelle du gène PI3, peptide antimicrobien bactéricide.
- d'assurer l'homéostasie énergétique de la peau en stimulant l'activité transcriptionnelle du gène ATP2C1 qui contribue à la formation d'ATP, molécule de stockage de l'énergie cellulaire utilisée pour assurer l'ensemble du métabolisme des cellules cutanées.
- d'améliorer et/ou renforcer la résistance de la peau aux stress en la protégeant des toxines par la stimulation de l'activité transcriptionnelle du gène GSTZ1, qui permet leur élimination par couplage avec la molécule de glutathion, tout en limitant l'inflammation par la stimulation de l'activité transcriptionnelle du gène PI3.

Ces résultats montrent que la composition cosmétique de l'invention permet de maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau, favoriser ses capacité adaptatives aux conditions d'hypoxie, renforcer le processus de détoxification et pondérer l'inflammation, diminuer la perte de fermeté, diminuer le relâchement des pores et la formation d'imperfections du relief cutané (rides et ridules), favoriser et/ou améliorer l'homogénéité de répartition d'un produit de maquillage, et fournit à la peau l'énergie nécessaire pour les peaux fatiguées et ternes et ainsi améliorer leur éclat.

### Exemple 2 : Formulations cosmétiques

Les formulations cosmétiques sont préparées selon les méthodes classiques de formulations. Les pourcentages sont exprimés en pourcentages pondéraux d'ingrédient (matière première) par rapport au poids total de la composition.

**Une crème de jour Hydratante pour peau mixte :**

| | |
|---|---|
| *GLYCEROL VEGETAL* (Glycerine) | 6.0% |
| FILMEXEL^{®} (*Caesalpinia spinosa fruit extract and Kappaphycus alvarezii extract and water)* | 0.50% |
| *ECOSKIN^{®}* (*Alpha-glucan oligosaccharide and Polymnia sonchifolia root juice and Maltodextrin and Lactobacillus)* | 0.50% |
| RENOHYAL (Sodium Hyaluronate) | 0.10% |
| Isononyl isononanoate | 10.0% |
| Steareth-2 | 12.5% |
| Glycéryl stearate | 1.1% |
| Alcool stéarylique | 5.0% |
| Butylène glycol | 3.0% |
| Glycérine | 2.0% |
| Conservateur | 0,5% |
| Eau, qsp | 100% |

**Un gel pour peau mixte :**

| | |
|---|---|
| *GLYCEROL VEGETAL (Glycerine)* | 6.0% |
| *ECOSKIN^{®} (Alpha-glucan oligosaccharide and Polymnia sonchifolia root juice and Maltodextrin and Lactobacillus)* | 0.50% |
| FILMEXEL^{®} (*Caesalpinia spinosa fruit extract and Kappaphycus alvarezii extract and water)* | 1.00% |
| RENOHYAL (Sodium Hyaluronate) | 0.1% |
| Glycol | 3.0% |
| Polymère d'AMPS | 3.0% |
| Huile minérale | 2.0% |
| Polyéthylène glycol | 1.5% |
| Conservateur | 0.5% |
| Concentré de parfum | 0,3% |
| Eau, Qsp | 100% |

**Un Fond de Teint**

| | |
|---|---|
| *ECOSKIN^{®}* (*Alpha-glucan oligosaccharide and Polymnia sonchifolia root juice and Maltodextrin* | |
| *and Lactobacillus)* | 0.50% |
| FILMEXEL^{®} (*Caesalpinia spinosa fruit extract and Kappaphycus alvarezii extract and water*) | 0.50% |
| OXYDES DE FER ET DIOXYDE DE TITANE (*Black Cl* 77499, *red Cl* 77491, *yellow Cl* 77492, *White Cl* 77891) | 18.0% |
| Cyclopentasiloxane et cyclohexasiloxane | 5.0% |
| Cetyl diméthicone | 1.0 % |
| Caprylic/capric triglycérides | 2.2 % |
| Octyl stéarate | 1.4 % |
| Huile minérale | 3.5 % |
| Cire d'abeille | 0.8 % |
| Polyméthacrylate de méthyle | 1.1% |
| Concentré de parfum | 0.1 % |
| Eau, qsp | 100% |

**Un Fond de Teint SPF**

| | |
|---|---|
| *BLUMILIGHT^{™} (Butylène glycol and Water and Theobroma cacao (cocoa) seed extract)* | 0.10% |
| FILMEXEL^{®} (*Caesalpinia spinosa fruit extract and Kappaphycus alvarezii extract and water)* | 0.50% |
| *ECOSKIN^{®} (Alpha-glucan oligosaccharide and Polymnia sonchifolia root juice and Maltodextrin and Lactobacillus)* | 0.50% |
| UV-TITANE M160 *(Titanium dioxide, TiO₂ (nano))* | 2.0% |
| OXYDE ZINC *(Zinc oxide, Cl* 77947) | 3.0% |
| OXYDES DE FER ET DIOXYDE DE TITANE (*Black Cl* 77499, *red Cl* 77491, *yellow Cl* 77492, *White Cl* 77891) | 16.0% |
| Cyclopentasiloxane et cyclohexasiloxane | 5.0% |
| Cetyl diméthicone | 1.0 % |
| Caprylic/capric triglycérides | 2.2 % |
| Octyl stéarate | 1.4 % |
| Huile minérale | 3.5 % |
| Cire d'abeille | 0.8 % |
| Polyméthacrylate de méthyle | 1.1% |
| Concentré de parfum | 0.1 % |
| Eau, qsp | 100% |

## Revendications

1. Composition cosmétique comprenant, dans un milieu physiologiquement acceptable au moins :
a) un agent cosmétique constitué par des galactomannanes de masses molaires comprises entre 5 et 630kDa, de préférence entre 5 et 120kDa, de préférence encore entre 8 et 80kDa, et des galactanes sulfatés réticulés de masses molaires comprises entre 7 et 3000kDa, de préférence entre 7 et 1100kDa, de préférence encore entre 8 et 200kDa, et
b) un prébiotique et un probiotique.

2. Composition cosmétique selon la revendication 1, **caractérisée** en ce l'agent cosmétique est constitué de galactomannanes obtenus par hydrolyse de galactomannanes natifs de Tara (*Caesalpinia spinosa),* de guar (*Cyamopsis tetragonoloba*)*,* de caroube (*Ceratonia siliqua*), mais aussi de séné (*Cassia angustifolia*), de cassier (*Cassia fistula*), de cassia (*Cassia obtusifolia* ou *Cassia tora*), de caroube chinois (*Gleditsia sinensis),* de févier (*Gieditsia triacanthos*), de sophora (*Sophora japonica*) et/ou de fenugrec (*Trigonella foenum-graecum*), de préférence de *Caesalpinia spinosa* ; et de galactanes sulfatés réticulés obtenus par hydrolyse de galactanes sulfatés natifs de carraghénanes (de *Kappaphycus alvarezii,* de *Kappaphycus striatum, d'Eucheuma cottonii, d'Eucheuma spinosum,* de *Chondrus crispus,* de *Gigartina skottsbergii,* de *Sacrothalia crsipata*), ou de *Fucellaria fastigiata,* d'Agar (*Gelidium sesquipedale)* ou des algues (*Polysiphonia lanosa* ou *Codium fragile),* de préférence de *Kappaphycus alvarezii.*

3. Composition cosmétique selon la revendication 1 ou 2, **caractérisée en ce que** l'agent cosmétique a) est constitué de 60 à 90% de galactomannanes et 10 à 40% de galactanes sulfatés réticulés.

4. Composition cosmétique selon l'une des revendications 1 à 3, **caractérisée en ce que** l'agent cosmétique est constitué de galactomannanes obtenus à partir de *Caesalpinia spinosa* de masses molaires comprises entre 1 et 150kDa, et de galactanes sulfatés réticulés obtenus à partir de *Kappaphycus alvarezii* de masses molaires comprises notamment entre 1 et 150kDa.

5. Composition cosmétique selon l'une quelconque des revendications 1 à 4, **caractérisée en ce que** l'agent cosmétique a) est présent dans la composition en une teneur allant 0,01% à 2% en poids de matière active par rapport au poids total de la composition, préférentiellement 0,1% à 0,1%, et préférentiellement encore de 0,4% à 0,8% en poids de matière active par rapport au poids total de la composition.

6. Composition cosmétique selon l'une quelconque des revendications 1 à 5, **caractérisée en ce qu'**elle comprend au moins, en tant que prébiotique, au moins un alpha-gluco- oligosaccharide et avantageusement en outre au moins un fructo-oligosaccharide, et en tant que probiotique, au moins un lysat du genre *Bacillus.*

7. Composition cosmétique selon la revendication 6, dans laquelle le prébiotique et le probiotique sont sous la forme d'un complexe pré-/pro-biotique comprenant un alpha-gluco- oligosaccharide, un extrait de racine de *Polymnia Sonchifolia* riche en fructo-oligosaccharide, un lysat de bactérie *Lactobacillus* et une maltodextrine.

8. Composition cosmétique selon l'une quelconque des revendications 1 à 7, **caractérisée en ce que** le prébiotique et le probiotique, en particulier sous la forme d'un complexe pré-/pro-biotique, sont présents dans la composition en une teneur totale en matière active allant de 0,05% à 3% en poids de matière active par rapport au poids total de la composition, préférentiellement de 0,1 à 1% en poids de matière active par rapport au poids total de la composition.

9. Composition cosmétique selon l'une quelconque des revendications 1 à 8, **caractérisée en ce qu'**elle se présente sous la forme d'un produit de soin et/ou d'un produit de maquillage pour la peau, les lèvres ou les yeux, en particulier pour le teint.

10. Procédé cosmétique de soin et/ou de maquillage des matières kératiniques, en particulier de la peau, des lèvres et/ou des yeux, comprenant au moins l'application sur lesdites matières kératiniques d'une composition cosmétique de soin et/ou de maquillage telle que définie dans l'une quelconque des revendications précédentes, en particulier d'une composition de maquillage pour le teint, les lèvres ou les yeux, de préférence pour le teint.

11. Procédé cosmétique de soin et/ou de maquillage de la peau selon la revendication 10, destiné à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier favoriser ses capacités adaptatives aux conditions d'hypoxie et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, favoriser l'homéostasie énergétique de la peau, et/ou favoriser et/ou améliorer la tenue du maquillage et l'homogénéité de son dépôt.

12. Utilisation cosmétique non thérapeutique d'au moins un agent cosmétique constitué par des galactomannanes de masses molaires comprises entre 5 et 630kDa et des galactanes sulfatés réticulés de masses molaires comprises entre 7 et 3000kDa , un prébiotique et une fraction probiotique tels que définis dans l'une quelconque des revendications 1 à 7, comme association destinée à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier favoriser ses capacités adaptatives aux conditions d'hypoxie et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, favoriser l'homéostasie énergétique de la peau, et/ou favoriser et/ou améliorer la tenue du maquillage et l'homogénéité de son dépôt.

## Patentansprüche

1. Kosmetische Zusammensetzung, die in einem physiologisch akzeptablen Medium mindestens umfasst:
a) ein kosmetisches Mittel, gebildet von Galactomannanen mit molaren Massen zwischen 5 und 630 kDa, vorzugsweise zwischen 5 und 120 kDa, weiterhin vorzugsweise zwischen 8 und 80 kDa, und vernetzten sulfatierten Galactanen mit molaren Massen zwischen 7 und 3000 kDa, vorzugsweise zwischen 7 und 1100 kDa, weiterhin vorzugsweise zwischen 8 und 200 kDa, und
b) ein Präbiotikum und ein Probiotikum.

2. Kosmetische Zusammensetzung nach Anspruch 1, **dadurch gekennzeichnet, dass** das kosmetische Mittel von Galactomannanen gebildet ist, die durch Hydrolyse von nativen Galactomannanen von Tara *(Caesalpinia spinosa),* von Guar (*Cyamopsis tetragonoloba*), von Johannisbrot (*Ceratonia siliqua*), aber auch von Senna *(Cassia angustifolia*), von Fisett-Cassie (*Cassia fistula*), von Senna obtusifolia (*Cassia obtusifolia* oder *Cassia tora*), von Chinesischer Gleditschie *(Gleditsia sinensis),* von Amerikanischer Gleditschie (*Gieditsia triacanthos*), von Japanischer Schnurbaum (*Sophora japonica*) und/oder von Bockshornklee (*Trigonella foenum-graecum*), vorzugsweise von *Caesalpinia spinosa;* erhalten sind, und von vernetzten sulfatierten Galactanen, die durch Hydrolyse von nativen sulfatierten Galactanen von Carrageenen (von *Kappaphycus aivarezii,* von *Kappaphycus striatum,* von *Eucheuma cottonii,* von *Eucheuma spinosum,* von *Chondrus crispus,* von *Gigartina skottsbergii,* von *Sacrothalia crsipata),* oder von *Fucellaria fastigiata,* von Agar *(Gelidium sesquipedale)* oder von Algen (*Polysiphonia lanosa* oder *Codium fragile),* vorzugsweise von *Kappaphycus aivarezii,* erhalten sind.

3. Kosmetische Zusammensetzung nach Anspruch 1 oder 2, **dadurch gekennzeichnet, dass** das kosmetische Mittel a) aus 60 bis 90% Galactomannanen und 10 bis 40% vernetzten sufatierten Galactanen gebildet ist.

4. Kosmetische Zusammensetzung nach einem der Ansprüche 1 bis 3, **dadurch gekennzeichnet, dass** das kosmetische Mittel aus Galactomannanen gebildet ist, die aus *Caesalpinia spinosa* mit molaren Massen zwischen 1 und 150 kDa erhalten sind, und aus vernetzten sulfatierten Galactanen, die aus *Kappaphycus alvarezii* mit molaren Massen insbesondere zwischen 1 und 150 kDa erhalten sind.

5. Kosmetische Zusammensetzung nach einem der Ansprüche 1 bis 4, **dadurch gekennzeichnet, dass** das kosmetische Mittel a) in der Zusammensetzung in einem Gehalt von 0,01 bis 2 Gew.-% Wirkstoff im Verhältnis zum Gesamtgewicht der Zusammensetzung, vorzugsweise von 0,1 bis 0,1 Gew.-%, und noch vorzugsweiser von 0,4 bis 0,8 Gew.-% Wirkstoff im Verhältnis zum Gesamtgewicht der Zusammensetzung vorhanden ist.

6. Kosmetische Zusammensetzung nach einem der Ansprüche 1 bis 5, **dadurch gekennzeichnet, dass** sie mindestens als Präbiotikum mindestens ein Alpha-gluco-Oligosaccharid und in vorteilhafter Weise ferner mindestens ein Fructo-Oligosaccharid und als Probiotikum mindestens ein Lysat der Spezies *Bacillus* umfasst.

7. Kosmetische Zusammensetzung nach Anspruch 6, wobei das Präbiotikum und das Probiotikum in Form eines prä-/probiotischen Komplexes vorliegen, umfassend ein Alpha-Gluco-Oligosaccharid, einen an Fructo-Oligosaccharid reichen Wurzelextrakt von *Polymnia Sonchifolia,* ein Lysat der Bakterie *Lactobacillus* und ein Maltodextrin.

8. Kosmetische Zusammensetzung nach einem der Ansprüche 1 bis 7, **dadurch gekennzeichnet, dass** das Präbiotikum und das Probiotikum, vor allem in Form eines prä-/probiotischen Komplexes, in der Zusammensetzung in einem Gesamtgehalt an Wirkstoff von 0,05 bis 3 Gew.-% Wirkstoff im Verhältnis zum Gesamtgewicht der Zusammensetzung, vorzugsweise von 0,1 bis 1 Gew.-% Wirkstoff im Verhältnis zum Gesamtgewicht der Zusammensetzung vorhanden sind.

9. Kosmetische Zusammensetzung nach einem der Ansprüche 1 bis 8, **dadurch gekennzeichnet, dass** sie in Form eines Pflegeprodukts und/oder eines Make-up-Produkts für die Haut, die Lippen oder die Augen, vor allem für den Teint, vorliegt.

10. Kosmetisches Pflege- und/oder Make-up-Verfahren von Keratinsubstanzen, vor allem der Haut, der Lippen und/oder der Augen, umfassend mindestens das Auftragen einer kosmetischen Pflege- und/oder Make-up-Zusammensetzung nach einem der vorangehenden Ansprüche, vor allem einer Make-up-Zusammensetzung für den Teint, die Lippen oder die Augen, vorzugsweise für den Teint, auf die Keratinsubstanzen.

11. Kosmetisches Pflege- und/oder Make-up-Verfahren der Haut nach Anspruch 10, das dazu bestimmt ist, das Ökosystem der Haut aufrechtzuerhalten und/oder das Gleichgewicht des Mikrobioms der Haut, vor allem die Mikrobenvielfalt, aufrechtzuerhalten, die Hautbarriere und ihren Hydrolipidfilm zu stärken, die Unversehrtheit und die Widerstandsfähigkeit der Haut gegenüber Stress, vor allem ihre Anpassungsfähigkeit an Hypoxiebedingungen, zu verbessern und/oder die Folgen einer hypoxischen Umwelt für die Haut zu begrenzen, die Schuppenbildung und/oder die Erneuerung der Epidermis zu fördern, den Verlust von Straffheit zu verringern, die Erschlaffung der Poren zu verringern, die Unregelmäßigkeiten auf der Oberfläche zu verringern, die Ebenmäßigkeit des Teints zu verbessern, die energetische Homöostase der Haut zu fördern und/oder die Haltbarkeit des Make-ups und die Gleichmäßigkeit seines Auftrags zu fördern und/oder zu verbessern.

12. Nicht therapeutische kosmetische Verwendung eines kosmetischen Mittels, gebildet aus Galactomannanen mit molaren Massen zwischen 5 und 630 kDa und vernetzten sulfatierten Galactanen mit molaren Massen zwischen 7 und 3000 kDa, einem Präbiotikum und einer Probiotikumfraktion nach einem der Ansprüche 1 bis 7 als Kombination, die dazu bestimmt ist, das Ökosystem der Haut aufrechtzuerhalten und/oder das Gleichgewicht des Mikrobioms der Haut, vor allem die Mikrobenvielfalt, aufrechtzuerhalten, die Hautbarriere und ihren Hydrolipidfilm zu stärken, die Unversehrtheit und die Widerstandsfähigkeit der Haut gegenüber Stress, vor allem ihre Anpassungsfähigkeit an Hypoxiebedingungen, zu verbessern und/oder die Folgen einer hypoxischen Umwelt für die Haut zu begrenzen, die Schuppenbildung und/oder die Erneuerung der Epidermis zu fördern, den Verlust von Straffheit zu verringern, die Erschlaffung der Poren zu verringern, die Unregelmäßigkeiten auf der Oberfläche zu verringern, die Ebenmäßigkeit des Teints zu verbessern, die energetische Homöostase der Haut zu fördern und/oder die Haltbarkeit des Make-ups und die Gleichmäßigkeit seines Auftrags zu fördern und/oder zu verbessern.

## Claims

1. Cosmetic composition comprising, in a physiologically-acceptable medium at least:
a) a cosmetic agent consisting of galactomannans of molar masses comprised between 5 and 630 kDa, preferably between 5 and 120 kDa, still more preferably between 8 and 80 kDa, and crosslinked sulfated galactans of molar masses comprised between 7 and 3000 kDa, preferably between 7 and 1100 kDa, still more preferably between 8 and 200 kDa, and
b) a prebiotic and a probiotic.

2. Cosmetic composition according to claim 1, **characterized in that** the cosmetic agent consists of galactomannans obtained by hydrolysis of native galactomannans from tara *(Caesalpinia spinosa),* guar (*Cyamopsis tetragonoloba*)*,* locust bean (*Ceratonia siliqua*), as well as senna (*Cassia angustifolia*), cassia (*Cassia fistula, Cassia obtusifolia* or *Cassia tora*), Chinese honey locust *(Gleditsia sinensis),* honey locust (*Gleditsia triacanthos*), Chinese scholar tree (*Sophora japonica*) and/or fenugreek (*Trigonella foenum-graecum*), preferably *Caesalpinia spinosa;* and crosslinked sulfated galactans obtained by hydrolysis of native galactans from carrageenans (*Kappaphycus alvarezii, Kappaphycus striatum, Eucheuma cottonii, Eucheuma spinosum, Chondrus crispus, Gigartina skottsbergii, Sacrothalia crispata),* or *Furcellaria fastigiata,* agar *(Gelidium sesquipedale)* or algae (*Polysiphonia lanosa* or *Codium fragile),* preferably *Kappaphycus alvarezii.*

3. Cosmetic composition according to claim 1 or 2, **characterized in that** cosmetic agent a) consists of from 60 to 90% of galactomannans and 10 to 40% of crosslinked sulfated galactans.

4. Cosmetic composition according to one of claims 1 to 3, **characterized in that** the cosmetic agent consists of galactomannans obtained from *Caesalpinia spinosa* of molar masses comprised between 1 and 150 kDa, and crosslinked sulfated galactans obtained from *Kappaphycus alvarezii* of molar masses comprised between 1 and 150 kDa, in particular.

5. Cosmetic composition according to any one of claims 1 to 4, **characterized in that** cosmetic agent a) is present in the composition in an amount ranging from 0.01% to 2% by weight of active ingredient relative to the total weight of the composition, preferentially 0.1% to 0.1%, and more preferentially still from 0.4% to 0.8% by weight of active ingredient relative to the total weight of the composition.

6. Cosmetic composition according to any one of claims 1 to 5, **characterized in that** it comprises at least, as prebiotic, at least one alpha-gluco-oligosaccharide and advantageously further at least one fructo-oligosaccharide, and as probiotic, at least one *Bacillus*-type lysate.

7. Cosmetic composition according to claim 6, wherein the prebiotic and probiotic are in the form of a pre/probiotic complex comprising an alpha-gluco-saccharide, a *Polymnia Sonchifolia* root extract rich in fructo-oligo-saccharide, a *Lactobacillus* bacterium lysate and a maltodextrin.

8. Cosmetic composition according to any one of claims 1 to 7, **characterized in that** the prebiotic and probiotic, in particular in the form of a pre-/probiotic complex, are present in the composition in a total content of active ingredient ranging from 0.05% to 3% by weight of active ingredient relative to the total weight of the composition, preferentially 0.1% to 1% by weight of active ingredient relative to the total weight of the composition.

9. Cosmetic composition according to any one of claims 1 to 8, **characterized in that** it is present in the form of a care and/or makeup product for the skin, lips or eyes, in particular for the complexion.

10. Cosmetic method for care and/or makeup for keratin materials, in particular skin, lips and/or eyes, comprising at least the application onto said keratin materials of a cosmetic care and/or makeup composition as defined according to any one of the preceding claims, in particular a makeup composition for the complexion, lips or eyes, preferably for the complexion.

11. Cosmetic care and/or makeup method for the skin according to claim 10, intended to maintain the skin ecosystem and/or maintain the balance of the skin microbiota, in particular microbial diversity, strengthen the skin barrier and its hydrolipidic film, improve the integrity and the resistance of the skin to stresses, in particular to promote its adaptive capacities to the conditions of hypoxia and/or limit the skin consequences of a hypoxic environment, promote desquamation and/or epidermal renewal, reduce the loss of firmness, reduce loss of pore elasticity, reduce surface irregularities, improve the uniformity of the complexion, promote the energetic homeostasis of the skin, and/or promote and/or improve makeup wear and the uniformity of its deposit.

12. Cosmetic nontherapeutic use of at least one cosmetic agent consisting of glucomannans of molar masses comprised between 5 and 630 kDa and crosslinked sulfated galactans of molar masses comprised between 7 and 3000 kDa, a prebiotic and a probiotic fraction such as defined according to any one of claims 1 to 7, as a combination intended to maintain the skin ecosystem and/or maintain the balance of the skin microbiota, in particular microbial diversity, strengthen the skin barrier and its hydrolipidic film, improve the integrity and the resistance of the skin to stresses, in particular to promote its adaptive capacities to the conditions of hypoxia and/or limit the skin consequences of a hypoxic environment, promote desquamation and/or epidermal renewal, reduce the loss of firmness, reduce loss of pore elasticity, reduce surface irregularities, improve the uniformity of the complexion, promote the energetic homeostasis of the skin, and/or promote and/or improve makeup wear and the uniformity of its deposit.
